# EUROPEAN PATENT APPLICATION

(11) **EP 1 967 224 A2**
(43) Date of publication of application: **10.09.2008**
(21) Application number: 08151317.8
(22) Date of filing: 12.02.2008
(51) Int. Cl.: A61M 16/10, B01D 53/047, C01B 13/02

(54) **Oxygen concentrator system**

(30) Priority: 27.02.2007 US 711527
(71) Applicant: Delphi Technologies, Inc., Troy, Michigan 48007 (US)
(72) Inventor: McClain, Michael S., Ortonville, MI 48462 (US); Pelletier, Dana, Ortonville, MI 48462 (US)
(74) Representative: Denton, Michael John

(57) **Abstract**

An oxygen concentrator system (10) includes a portable system (14) having a portable oxygen concentrator with a portable compressor (36). The portable system further includes a power supply (26), a control system (30), and a portable oxygen outlet (34) configured to selectively provide a first direct fluid supply. A station (18) is configured to engage the portable system. The station may be selectively controlled by the control system (30) when engaged with the portable system. The station may have a stationary oxygen concentrator that is inoperable for delivering a second direct fluid supply via a stationary oxygen outlet during delivery of the first direct fluid supply via the portable oxygen outlet when the station and portable system are engaged. Further, the portable oxygen concentrator is inoperable for delivering the first direct fluid supply via the portable oxygen outlet during delivery of the second direct fluid supply via the stationary oxygen outlet when the station and portable system are engaged.

## Description

### TECHNICAL FIELD

The present disclosure relates generally to oxygen generators, and more particularly to a system for selectively docking a portable oxygen concentrator.

### BACKGROUND OF THE INVENTION

Relatively light and portable oxygen concentrators are generally not adapted to produce the high oxygen output that is periodically required by many users, particularly at night. Conversely, oxygen concentrators adapted for high output oxygen production tend to be larger and/or heavier than those adapted for portable use. As such, an oxygen concentrator user who requires a portable oxygen concentrator as well as periodic high oxygen output often compromises with either a relatively heavy transportable device, or alternates between two oxygen concentrators (one adapted for portable use and the other adapted for high oxygen output for stationary use).

As such, a need exists for an oxygen concentrator system that provides for a relatively lightweight and portable design and the ability to selectively produce high oxygen output for an extended period of time.

### SUMMARY OF THE INVENTION

An oxygen concentrator system includes a portable system having a portable oxygen concentrator with a portable compressor. The portable system further includes a power supply, a control system, and a portable oxygen outlet configured to selectively provide a first direct fluid supply. A station is configured to engage the portable system. The station is selectively controlled by the control system when engaged with the portable system. The station may have a stationary oxygen concentrator that is inoperable for delivering a second direct fluid supply via a stationary oxygen outlet during delivery of the first direct fluid supply via the portable oxygen outlet. Further, the portable oxygen concentrator is inoperable for delivering the first direct fluid supply via the portable oxygen outlet during delivery of the second direct fluid supply via the stationary oxygen outlet.

### BRIEF DESCRIPTION OF THE DRAWINGS

Objects, features and advantages of embodiments of the present disclosure will become apparent by reference to the following detailed description and drawings, in which like reference numerals correspond to similar, though not necessarily identical components. Reference numerals having a previously described function may not necessarily be described in connection with other drawings in which they appear.

Fig. 1 is a schematic perspective view of an embodiment of an oxygen concentrator system showing a portable system and a system-engaging station exploded away; and

Fig. 2 is a partial system block diagram of the oxygen concentrator system of Fig. 1.

### DESCRIPTION OF THE PREFERRED EMBODIMENT

Embodiment(s) of the oxygen concentrator system disclosed herein advantageously include a portable system that is selectively engageable with a system-engaging station, whereby the oxygen concentrator system may provide for both ambulatory and stationary use. The portable system may include the necessary components to deliver oxygen to a patient independent of the station, whereas the station may be configured to provide for increased oxygen output when the portable system is engaged therewith. As such, the station may advantageously eliminate the need to carry the components required for higher oxygen output when the system is in ambulatory mode. The increased oxygen output adapted to be provided by the station may be appropriate for increased prescription settings and/or for nighttime use.

Embodiment(s) of the oxygen concentrator system disclosed herein may also be advantageously smaller, lighter, and/or more power-efficient than previous stationary and/or ambulatory systems (including combined systems), while maintaining or exceeding performance expectations. As such, one or more components of the portable system may be configured for portability and/or moderate oxygen output, whereas one or more components of the station may be configured for stationary use and/or higher-volume oxygen output. It is to be understood that although the portable system and station may be configured for moderate oxygen output and high-volume oxygen output, respectively, they may provide lower oxygen volumes if desired.

It is to be further understood that "oxygen," as used herein, is intended to be interpreted broadly and may include pure oxygen, as well as a gaseous mixture including oxygen.

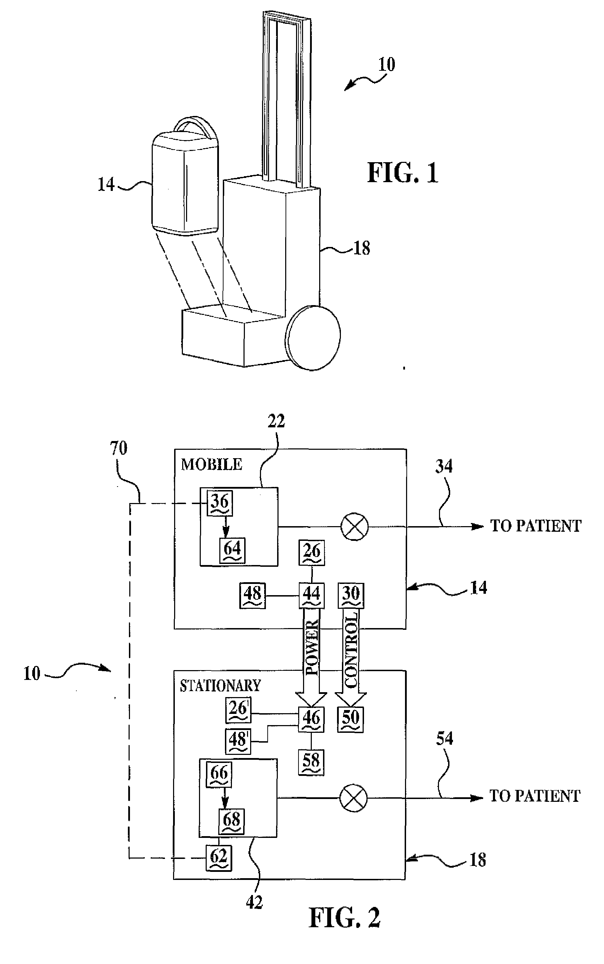

Referring now to Figs. 1 and 2 together, an embodiment of the oxygen concentrator system 10 includes a portable system 14, and a station 18 configured to engage the portable system 14. The portable system 14 may include a portable oxygen concentrator 22, a power supply 26, a power interface 44, a control system 30, and/or a portable oxygen outlet 34, which portable oxygen outlet 34 may be configured to selectively provide a direct fluid supply. In an embodiment, the portable oxygen concentrator 22 may include a portable compressor 36.

It is to be understood, as used herein, that a "direct fluid supply" refers to a fluid supply that is not delivered from a storage tank, but, rather, is output without substantial delay after being generated and/or harnessed. An example of such a direct fluid supply is an oxygen-rich gas flowing from the oxygen concentrator 22 via a substantially unobstructed passageway to an oxygen outlet 34, where it may then be provided to a patient.

The portable system 14 may be configured to be lightweight and/or portable. In an embodiment, the weight of the portable system 14 is equal to or less than about 10 Ibs, and has a general volumetric size of less than about 400 in³. In a non-limiting embodiment, this general volumetric size ranges from about 200 in³ to about 400 in³. As such, the portable system 14 may include one or more components configured to be lightweight, some examples of which include the portable oxygen concentrator 22; the portable compressor 36; the power supply 26; the control system 30; one or more valves, which may be included in, or may interconnect any of the portable system 14 components and/or subcomponents; and/or one or more electronic components, which also may be included in, or may interconnect any of the portable system 14 components and/or subcomponents. In an embodiment, a component may be configured to be lightweight by design and/or composition. As non-limitative examples, a component may be designed to use fewer and/or smaller subcomponents to reduce component weight, and/or may be fabricated from one or more relatively lightweight materials.

Referring now more particularly to Fig. 2, in an embodiment, the power supply 26 includes at least one battery, which may be single-use, rechargeable, or combinations thereof. The system 10 may also include a power supply recharging device 48, which may be engaged with the portable system 14. In an embodiment, the power supply recharging device 48 is a DC battery charger and, thus, may be configured to recharge a battery when the power supply recharging device is in communication with a DC power source. The power supply recharging device 48 may be in operative communication with the power interface 44 and/or the power supply 26. In one embodiment, the power supply recharging device 48 is located within the portable system 14, and in another embodiment, the power supply recharging device 48 is independent from the portable system 14, but is in electric communication therewith.

The station 18 may be selectively controlled by the control system 30. As such, in an embodiment, the station 18 is operable when the portable system 14 is engaged therewith. In other words, one oxygen concentrator system (either in the portable system 14 or the station 18) is active at a time when the station 18 is engaged with the portable system 14, and the patient receives the direct fluid supply from the active system (e.g., via portable oxygen outlet 34 or via stationary oxygen outlet 54 (discussed further below)).

Station 18 includes a stationary oxygen concentrator 42. In an embodiment, the stationary oxygen concentrator 42 is inoperable during operation of the portable oxygen concentrator 22 when the portable system 14 is engaged with the station 18. In another embodiment, the stationary oxygen concentrator 42 is operable when the portable system 14 is engaged with the station 18. In still another embodiment, the operator may choose between the portable oxygen concentrator 22 and the stationary oxygen concentrator 42 when the portable system 14 is docked in the station 18. Further, it is to be understood that the oxygen concentrator system 10 may be programmed to have either the stationary oxygen concentrator 42 or the portable oxygen concentrator 22 operate when the portable system 14 is engaged with the stationary system 18, as desired.

The station 18 may have a power jack 46 configured to selectively conduct power to the power supply 26. The system 10 may also include a second or alternate power supply recharging device 48', which may be engaged with the station 18. The power supply recharging device 48' may be configured to selectively recharge the power supply 26 when the portable system 14 is engaged with the station 18. In an embodiment, the power supply recharging device 48' is a DC battery charger and, thus, may be configured to recharge a battery when the power supply recharging device is in communication with a DC power source. The power supply recharging device 48' may be in operative communication with the power jack 46 and/or the power supply 26. In one embodiment, the power supply recharging device 48' is located within the station 18, and in another embodiment, the power supply recharging device 48' is independent from the station 18 but is in electric communication therewith.

The station 18 may also include a control inlet 50, which may be in operative electrical communication with the power jack 46 and/or the stationary oxygen concentrator 42. The control inlet 50 may be configured to selectively receive one or more controls from the control system 30. As such, the control system 30 may be configured to selectively control operation of the portable system 14 and/or the station 18. As a non-limitative example, the control system 30 may be configured to provide one or more commands to the portable system 14 and/or to the station 18. It is to be understood that the commands may be configured to trigger the portable system 14 and/or the station 18 to, for example, initiate operation, increase oxygen volume, decrease oxygen volume, cease operation, and/or any other commands, as desired.

The station 18 also includes a stationary oxygen outlet 54, which is in operative communication with the stationary oxygen concentrator 42 and is configured to selectively provide a direct fluid supply. In an embodiment, the station 18 is configured to selectively provide high oxygen output via the stationary oxygen outlet 54. As defined herein, "oxygen output" refers to the delivery of oxygen to a patient from the portable system 14 and/or the stationary system 18. Suitable forms of oxygen output include, but are not limited to, oxygen flows, oxygen pulses, oxygen boluses, or combinations thereof. In a non-limiting example, the high oxygen output ranges from about 0.5 standard liters per minute (slpm) to about 8.0 slpm of continuous output. The low oxygen output is generally a pulse-delivered bolus. As alluded to hereinabove, it is to be understood that the portable oxygen concentrator 22 and the stationary oxygen concentrator 42 are configured such that they 22, 42 do not output fluid through a shared outlet. Rather, each provides a direct fluid supply through the respective outlet 34, 54.

It is to be understood that in an embodiment, the stationary oxygen concentrator 42 receives control via the control inlet 50. As such, the station 18, including the stationary oxygen concentrator 42 and the stationary oxygen outlet 54, are operable when the portable system 14 is engaged with the station 18. In another embodiment, power and control are shared between the portable system 14 and the station 18, wherein the portable system 14 and the station 18 each independently embodies all other components necessary for providing a fluid having a high oxygen concentration.

In an embodiment, an electrical converter 58 is in operative communication with the station 18 and is configured to convert electricity flowing to the portable system 14 and/or the station 18 from AC (alternating current) to DC (direct current). The electrical converter 58 may be in operative communication with the power jack 46 and/or the power supply recharging device 48, 48'. It is to be understood that the electrical converter 58 may be configured to allow the system 10 (including the portable system 14 and/or the station 18) to operate off of either AC, such as, for example, electric power from a standard electrical outlet, or DC, such as, for example, accessory power from an automobile or boat. In an embodiment, the portable system 14 is configured to independently operate off of DC, and the system 10, including both the portable system 14 and the station 18, is configured to operate off of AC.

A spare power supply 26' may be removably engaged with the stationary system 18 and charged by the power supply recharging device 48'. If the power supply 26 becomes depleted, the spare power supply 26' may be used to extend the duration of operation of the portable system 14.

As mentioned above, the portable oxygen concentrator 22 is inoperable during operation of the stationary oxygen concentrator 42 when the portable system 14 is engaged with the station 18. Alternately, when the portable system 14 is engaged with the station 18, the stationary oxygen concentrator 42 is inoperable during operation of the portable oxygen concentrator 22 (i.e., the patient may select between the portable system 14 and the station 18 while the two are docked together). In yet another embodiment, when the portable system 14 is not engaged with the station 18, the stationary oxygen concentrator 42 is inoperable.

Alternately, when the portable system 14 and station 18 are not engaged (not docked together), it is to be understood that each of the concentrators 22, 42 may be simultaneously operating, if desired; in this embodiment, the stationary system 18 may have its own control system (not shown), similar to the control system 30 of the portable system 14.

The station 18, including the stationary oxygen concentrator 42, may be configured to provide increased fluid flow, as compared to fluid flow provided by the portable system 14 utilizing the portable oxygen concentrator 22. Station 18 may be configured to provide fluid flow, including increased fluid flow, for extended and generally non-limited periods of time since the station 18 is plugged into the power supply 26', which is a relatively continuous source of power. "Increased fluid flow" is to be interpreted broadly and may include an amount of oxygen output by the system 10 utilizing the station 18, which may be greater than the amount of oxygen output by the system 10 utilizing only the portable system 14. Further, "increased fluid flow" or "oxygen-rich gas" may be defined as a predetermined oxygen level of the output fluid and/or a predetermined oxygen output rate (i.e., the volume of oxygen output during a predetermined time period). As non-limitative examples, increased fluid flow may be preferred for increased prescription settings and/or for nighttime use. It is to be understood that any suitable embodiment(s) of the station 18 and/or the stationary oxygen concentrator 42 may be utilized to provide a desired fluid output.

The portable oxygen concentrator 22 and/or the stationary oxygen concentrator 42 may include any suitable devices and/or configurations to generate the desired oxygen-rich gas. As a non-limitative example, the portable oxygen concentrator 22 and/or the stationary oxygen concentrator 42 may include a respective pressure swing adsorption (PSA) system or a vacuum pressure swing adsorption (VPSA) system 64, 68. In an embodiment, the PSA or VPSA system 64, 68 includes a PSA or VPSA sieve bed, valves, fittings, manifolds, tubing, pressure sensors, oxygen sensors, filters, and/or combinations thereof.

In an embodiment, the portable oxygen concentrator 22 and/or the stationary oxygen concentrator 42 include a compressor, such as portable compressor 36 and stationary compressor 66. The respective compressors 36, 66 take ambient gas (i.e., air), pressurize the gas, and deliver the pressurized gas to the respective PSA or VPSA system 64, 68. It is to be understood that when a VPSA system is used, means for drawing a vacuum on the pressurized gas is also included. A non-limiting example of such means is a vacuum pump.

In a further embodiment, a motor 62 is engaged with the station 18 and additionally may be configured to selectively couple with (indicated at dashed line 70 in Fig. 2) and overdrive the portable compressor 36, whereby the compressed air may be delivered to the stationary oxygen concentrator 42. Overdriving the portable compressor 36 may result in increased fluid flow from the portable compressor 36, as compared with the fluid flow achieved without overdriving the portable compressor 36. As such, the motor 62 may provide for the desired fluid pressure and flow to realize increased fluid flow, as defined hereinabove. In an embodiment, the motor 62 may be packaged with the electrical converter 58, the power supply recharging device 48', the spare power supply 26', and/or additional electronics, if desired.

As previously indicated, the stationary oxygen concentrator 42 may also include stationary compressor 66. The stationary compressor 66 may be configured to selectively provide a compressed fluid to the stationary oxygen concentrator 42 when the portable system 14 is engaged with the station 18. The stationary compressor 66 may be configured to automatically become the default compressor when the portable system 14 is engaged with station 18. The stationary compressor 66 may provide the desired fluid pressure and flow to realize increased fluid flow, as defined hereinabove. In an embodiment, the stationary compressor 66 may be packaged with the electrical converter 58, the power supply recharging device 48', the spare power supply 26', and/or additional electronics, if desired.

In another embodiment, the stationary concentrator 42 may be connected to the compressor 36 of the portable system 14 when the portable system 14 is docked. It is to be understood that, in this embodiment, the stationary system 18 may be configured without its own compressor, but could operatively use the compressor 36 of the portable system 14. It is also to be understood that (as mentioned above), in a non-limiting embodiment, the stationary system 18 may be inoperable unless the stationary system 18 and the portable system 14 are connected.

It is to be understood, as used herein, that "operable" is to be interpreted broadly and may refer to a condition of being able to operate. As such, "operable" infers that a device or component may be utilized. In an embodiment, "operable" does not necessitate that the device or component be utilized during all times of operability. As an example, the stationary oxygen concentrator 42 may be operable even if it is turned off (and, thus, not currently operating). Further, "inoperable" as used herein is meant to indicate that the respective system 14/station 18 is not delivering oxygen to the patient, although one or more components of the "inoperable" respective system 14/station 18 may be concurrently functioning.

Further, it is to be understood that the terms "engaged/engage/engaging", in "communication" with and/or the like are broadly defined herein to encompass a variety of divergent connected arrangements and assembly techniques. These arrangements and techniques include, but are not limited to (1) the direct communication between one component and another component with no intervening components therebetween; and (2) the communication of one component and another component with one or more components therebetween, provided that the one component being "engaged with" the other component is somehow in operative communication with the other component (notwithstanding the presence of one or more additional components therebetween). For example, the portable system 14 may be engaged with the station 18 although other components are disposed therebetween.

While several embodiments have been described in detail, it will be apparent to those skilled in the art that the disclosed embodiments may be modified. Therefore, the foregoing description is to be considered exemplary rather than limiting.

## Claims

1. An oxygen concentrator system, comprising:
a portable system including a portable oxygen concentrator having a portable compressor; a power supply; a control system; and a portable oxygen outlet configured to selectively provide a first direct fluid supply; and
a station configured to engage the portable system, the station being selectively controlled by the control system when engaged with the portable system, the station having a stationary oxygen concentrator that is inoperable for delivering a second direct fluid supply via a stationary oxygen outlet during delivery of the first direct fluid supply via the portable oxygen outlet;
wherein the portable oxygen concentrator is inoperable for delivering the first direct fluid supply via the portable oxygen outlet during delivery of the second direct fluid supply via the stationary oxygen outlet.

2. The oxygen concentrator system of claim 1 wherein the control system is configured to selectively control operation of the portable system or the station.

3. The oxygen concentrator system of claim 1 wherein the power supply includes at least one of a battery or a rechargeable battery.

4. The oxygen concentrator system of claim 1, further comprising a power supply recharging device engaged with the station and configured to selectively recharge the power supply when the portable system is engaged with the station.

5. The oxygen concentrator system of claim 1, further comprising an electrical converter in operative communication with the station and configured to convert electricity from AC to DC.

6. The oxygen concentrator system of claim 1, further comprising a motor engaged with the station and configured to selectively couple with and overdrive the portable compressor to provide a compressed fluid to the stationary oxygen concentrator.

7. The oxygen concentrator system of claim 1, wherein the stationary oxygen concentrator includes a stationary oxygen compressor configured to selectively provide a compressed fluid to the stationary oxygen concentrator when the portable system is engaged with the station.

8. The oxygen concentrator system of claim 1 wherein the stationary oxygen concentrator is operable when the portable system is engaged with the station.

9. The oxygen concentrator system of claim 1 wherein at least one of the portable oxygen concentrator or the stationary oxygen concentrator includes a pressure swing adsorption system.

10. The oxygen concentrator system of claim 1 wherein the portable system includes at least one component configured to be lightweight.

11. The oxygen concentrator system of claim 10 wherein the at least one component is selected from the portable compressor, the portable oxygen concentrator, the power supply, the control system, one or more valves, one or more electronic components, and combinations thereof.

12. The oxygen concentrator system of claim 1 wherein the station is configured to selectively provide high oxygen output via the stationary oxygen outlet.

13. A station configured for engagement with a portable system, the portable system comprising:
a portable oxygen concentrator having a portable compressor;
a portable power supply;
a control system; and
a portable oxygen outlet configured to selectively provide a first direct fluid supply, wherein the station includes:
a power jack configured to selectively conduct power to the power supply;
a control inlet in operative electrical communication with the power jack and configured to selectively receive one or more controls from the control system; and
a stationary oxygen concentrator in operative electrical communication with the control inlet, the stationary oxygen concentrator inoperable for delivering a second direct fluid supply via a stationary oxygen outlet during delivery of the first direct fluid supply via the portable oxygen outlet, the stationary oxygen outlet being in fluid communication with the stationary oxygen concentrator;
wherein the portable oxygen concentrator is inoperable for delivering the first direct fluid supply via the portable oxygen outlet during delivery of the second direct fluid supply via the stationary oxygen outlet.

14. The station of claim 13, further comprising a motor configured to couple with and selectively overdrive the portable compressor to provide a compressed fluid to the stationary oxygen concentrator.

15. The station of claim 13 wherein the stationary oxygen concentrator includes a stationary compressor configured to selectively provide a compressed fluid to a pressure swing adsorption system in the stationary oxygen concentrator when the portable system is engaged with the station.

16. The station of claim 13 wherein the stationary oxygen concentrator is operable when the portable system is engaged with the station.

17. The station of claim 13 wherein at least one of the stationary oxygen concentrator or the portable oxygen concentrator includes a pressure swing adsorption system, a vacuum pressure swing absorption system, or a combination thereof.

18. The station of claim 13, further comprising an electrical converter in operative communication with the power jack and configured to selectively convert electricity from AC to DC.

19. The station of claim 13, further comprising a power supply recharging device in operative communication with the power jack and configured to selectively recharge the portable power supply when the portable system is engaged with the station.
